# EUROPEAN PATENT SPECIFICATION

(11) **EP 3 296 741 B1**
(45) Date of publication and mention of the grant of the patent: **21.08.2019**
(21) Application number: 17190753.8
(22) Date of filing: 13.09.2017
(51) Int. Cl.: G01N 33/538, G01N 33/569

(54) **MULTIPLEX BEAD BASED ASSAY FOR QUANTIFICATION OF MULTIPLE TYPES OF CARRIER PROTEIN IN A MULTIVALENT CONJUGATE VACCINE COMPOSITION**
MULTIPLEX-BEAD-TEST ZUR QUANTIFIZIERUNG MEHRERER ARTEN VON TRÄGERPROTEIN IN EINER MULTIVALENTEN KONJUGATIMPFSTOFFZUSAMMENSETZUNG
ESSAI À BASE DE BILLES MULTIPLEXES POUR LA QUANTIFICATION DE PLUSIEURS TYPES DE PROTÉINES PORTEUSES DANS UNE COMPOSITION VACCINALE CONJUGUÉE MULTIVALENTE

(30) Priority: 14.09.2016 IN 201621031359
(43) Date of publication of application: 21.03.2018
(73) Proprietor: Serum Institute of India Private Limited, 411028 Maharastra (IN)
(72) Inventor: MHALASAKANT, Dhere Rajeev, 411028 Maharastra (IN); DINESH, Mallya Asha, 411028 Maharastra (IN); JAGDISHBHAI, Soni Dipen, 411028 Maharastra (IN); ISMAIL, Sayyed Mohsin, 411028 Maharastra (IN)
(74) Representative: Ström & Gulliksson AB

(56) References cited:
- EP-A1- 2 977 759
- WO-A1-2009/148395

## Description

### FIELD

The present disclosure relates to estimation of carrier proteins, used in conjugate vaccines. The scope of protection is defined by the appended claims.

### BACKGROUND

Isolated bacterial capsular polysaccharides from Haemophilus influenzae, Streptococcus pneumoniae, and Neisseria meningitidis have been successfully used as vaccines in adults. Vaccines based on capsular polysaccharides stimulate humoral immunity but fail to induce immunologic memory, and thus are ineffective in young infants.

For a vaccine to be effective and induce long lasting immunity, induction of T cell memory is very important. Increased immunogenic response of an antigen can be achieved by converting the T-independent antigen to T-dependent antigen. The conjugation of polysaccharide to carrier protein renders the antigenic molecule to undergo the process of antigen presentation, and enhance the polysaccharide immunogenicity by eliciting the T-cell dependent response. The proteins used for conjugation to polysaccharides include CRM197, tetanus toxoid, diphtheria toxoid, Neisseria meningitidis outer membrane complex, Haemophilus influenzae protein D, Pneumolysin, etc.

The meningococcal meningitis is caused by Neisseria meningitidis (meningococcus) which is an aerobic Gram-negative encapsulated bacterium. To date, more than 10 serotypes of meningococcus have been characterized by differences in the polysaccharide capsule. The polysaccharides from serotype A,C,W,Y & X have been conjugated to various carrier proteins to prepare a conjugated vaccine, effective against infection by these serotypes.

Similarly for Streptococcus pneumoniae, more than 90 distinct serotypes have been identified throughout the world (WHO); of which a small number of serotypes accounts for most diseases in infants. Pneumococcal conjugate vaccines containing poly-saccharides from 7+ serotypes i.e. Prevnar & Synflorix are already into market. The polysaccharides from Pneumo serotype have been conjugated to various carrier proteins i.e. CRM197, Protein D, Tetanus toxoid (TT), Diphtheria toxoid (DT), etc. to prepare a conjugated Ps-Pr vaccine against these serotypes.

To increase the coverage of these vaccines across serotypes for a bacterium, conjugated polysaccharide from various serotypes is added to vaccine composition, inadvertently increasing the concentration of carrier protein. It is observed that coadministration of conjugate vaccines bearing the same carrier protein, reduces the immune response (due to immune interference) against polysaccharides conjugated with that particular protein [Ref: "Comparative effects of carrier proteins on vaccine-induced immune response" by Knuf M. et al; Vaccine. 2011 Jul 12;29(31):4881-90]. Also, it has been observed that the combination of polysaccharide from a particular serotype with a specific carrier protein shows better efficacy. [Ref: "Comparison of CRM197, diphtheria toxoid and tetanus toxoid as protein carriers for meningococcal glycoconjugate vaccines." by Tontini M. et al; Vaccine. 2013 Oct 1; 31(42):4827-33].

To circumvent the phenomenon of negative interference in multivalent vaccines composed of polysaccharide-protein conjugates and to provide better immune response, several vaccine manufacturers are developing vaccines comprising multiple carrier proteins conjugated to different polysaccharides so that the maximum load of each of the carrier proteins is not reached.

Synflorix, a licensed 10 valent Pneumococcal polysaccharide conjugate vaccine comprises Ps conjugated to three different carrier proteins. Polysaccharide from serotype 1, 4, 5, 6B, 7F, 9V, 14 and 23F are conjugated to protein D (derived from non-typeable Haemophilus influenzae) carrier protein; Polysaccharide from serotype 18C is conjugated to tetanus toxoid carrier protein; and polysaccharide from serotype 19F is conjugated to diphtheria toxoid carrier protein. A 11 valent Pneumococcal conjugate vaccine comprising Ps conjugated to at least two different carrier proteins has also been reported (Ref: Rose-Marie Olandera et al 2002; Booster response to the tetanus and diphtheria toxoid carriers of 11-valent pneumococcal conjugate vaccine in adults and toddlers; Vaccine 20; 336-341) wherein polysaccharide is selected from 1, 3, 4, 5, 6B, 7F, 9V, 14, 18C, 19F and 23F and carrier proteins are Diphtheria toxoid (DT) and tetanus toxoid (TT). Also, a pentavalent Meningococcal conjugate vaccine comprising of Ps conjugated to two different carrier proteins is being developed, wherein polysaccharide from serotype A and X are individually conjugated to tetanus toxoid and polysaccharide from serotype C,Y,W are individually conjugated to CRM197 (Ref: S S Pisal et al; Immunogenicity of the Meningitis Vaccine Project's pentavalent Men ACWYX polysaccharide conjugate vaccine MCV-5; Abstract ID: 035; 20th International Pathogenic Neisseria Conference, 4th-9th September 2016. Manchester, United Kingdom).

According to the WHO's recommendations for the production & control of pneumococcal conjugate vaccines, the total protein content of the conjugate should be determined by means of an appropriately validated assay. [Ref: 54th meeting of the WHO Expert Committee on Biological Standardization, 17-21 November 2003]. Similarly, WHO's Recommendations to assure the quality, safety and efficacy of group A meningococcal conjugate vaccines require the label on the carton or the leaflet accompanying the container to indicate the amounts of group A meningococcal saccharide and carrier protein contained in each single human dose [Ref: WHO Expert Committee on Biological Standardization Fifty-seventh report; WHO Technical Report Series No. 962, 2011].

Techniques for estimation of protein concentration in a sample are very well known in the art. The physio-chemical techniques like Lowry method, Bradford method, Biuret method etc. and immunological techniques like ELISA are the most widely used assays for the estimation of protein concentration.

### Physico-chemical Methods:

Lowry's assay is the most widely used assay for determination of total protein in a sample. The method is a sensitive colorimetry based assay employing two color forming reaction. The amount of color produced in this assay by any given protein (or mixture of proteins) is dependent on the amino acid composition of the protein(s). In cases where in two or more proteins are present in a sample, negative interference may result in inaccurate determination of protein concentration [Ref: The Lowry Method for Protein Quantitation by Jakob H. Waterborg]. Also, the individual concentration of the different proteins in a sample cannot be determined using physico-chemical methods.

### Bio-chemical Methods:

WO 2009/148395 A1 discloses a method, which is suitable for quantifying virus or virus antigen in vaccine production, both in process samples and final vaccine samples. Enzyme linked immunosorbent assay (ELISA) is a biochemical technique used in immunology to detect the presence of proteins or antigens in a sample. In ELISA, analyte to be detected is fixed to a surface and then specific antibody linked to an enzyme is applied. In a later step this enzyme converts to some detectable signal i.e. color change in a sample. Although ELISA is a highly sensitive assay, it requires a relatively high amount of sample, especially in cases requiring detection and quantification of multiple proteins in a sample. Being a user dependent method, it suffers reproducibility issues and requires long time to execute the assay. The other disadvantages of ELISA include instability of the linked enzymes, relatively expensive substrates and require multiple steps to execute, all of which lead to relatively high costs for ELISA tests. Performing ELISAs for multiple antigens or multiple antibodies in vaccine evaluation is a time consuming and laborious job. There is a need to develop more efficient immunoassays.

Physiochemical methods like Lowry assay, Bradford method, etc. are able to determine the total concentration of protein in a sample, but fail to estimate individual concentration of the different proteins in a mixed sample. Biochemical method like ELISA is highly sensitive immunological assay but is relatively expensive, time consuming and laborious. There is need for a rapid and convenient alternative assay for estimation of individual concentration of carrier proteins in a multivalent polysaccharide protein conjugate vaccine composition wherein said composition comprises of at least 2 different types of carrier protein.

### OBJECTS

Some of the objects of the present disclosure, which at least one embodiment herein satisfies, are as follows:
An object of the present disclosure is to ameliorate one or more problems of the prior art or to at least provide a useful alternative.

Another object of the present disclosure is to provide a method to detect individual concentration of multiple carrier proteins.

Still another object of the present disclosure is to provide a simple, rapid and cost-effective method for detecting individual concentration of multiple carrier proteins.

Yet another object of the present disclosure is to provide a method for detecting individual concentration of multiple carrier proteins, which generates reproducible results.

Other objects and advantages of the present disclosure will be more apparent from the following description, which is not intended to limit the scope of the present disclosure.

### SUMMARY:

The present disclosure relates to a rapid and convenient method of estimation of individual concentration of at least 2 different types of carrier proteins in a sample comprising of multivalent polysaccharide protein conjugate, wherein said sample preferably is a vaccine composition or bulk.

The present method is a multiplex bead based competitive inhibition assay used for estimation of carrier protein concentration in a vaccine composition on the basis of fluorescence signal measured by suspension array system.

### Brief Description of the Drawings

**Figure: 1A** Standard curve for estimation of Carrier Protein CRM197 in MCV5 composition
**Figure: 1B** Standard curve for estimation of Carrier Protein TT in MCV5 composition
**Figure: 2A** Standard curve for estimation of Carrier Protein CRM197 in PCV composition
**Figure: 2B** Standard curve for estimation of Carrier Protein TT in PCV composition
**Figure: 2C** Standard curve for estimation of Carrier Protein DT in PCV composition
**Figure: 3A** CRM197 Multiplexed BBCIA Parallelism analysis in MCV5 composition to determine assay suitability
**Figure: 3B** TT Multiplexed BBCIA Parallelism analysis in MCV5 composition to determine assay suitability
**Figure: 4A** Accuracy of Multiplexed BBCIA for CRM/TT estimation in MCV5 formulation
**Figure: 4B** Accuracy of Multiplexed BBCIA for CRM/TT/DT estimation in PCV formulation
**Figure: 5** Robustness of Multiplexed BBCIA for CRM/TT estimation in MCV5 formulation
**Figure: 6** Specificity of Multiplexed BBCIA for CRM/TT estimation in MCV5 formulation

### DESCRIPTION:

According to World Health Organization, the vaccine manufacturer must determine and mention the total concentration of carrier protein, free and conjugated polysaccharide available in final vaccine formulation by using a properly validated assay.

The present disclosure relates to a multiplex bead based competitive inhibition assay to be used for estimation of individual carrier protein concentration in a vaccine composition on the basis of fluorescence signal measured by suspension array system i.e. Bioplex200 system comprising following steps:
a) Beads were coloured internally with two different fluorescent dyes (red and infrared). Ten different concentrations of red and infrared dyes were used to generate distinct bead regions. Each bead region was covalently coupled to respective carrier protein;
b) Diluting vaccine formulation to a suitable standard concentration to comply with the multiplexed bead based competitive inhibition assay limits;
c) Adding the anti-carrier protein antibody to the vaccine formulation containing multivalent polysaccharide-protein conjugates, free polysaccharide and free carrier protein;
d) Adding the vaccine formulation of step (c) containing free or unbound anti-carrier protein antibody, anti-carrier protein antibody bound to free and conjugated carrier protein, and free polysaccharide to carrier protein coupled beads;
e) The above mixture was washed using assay buffer followed by aspiration using vacuum manifold;
f) Adding the fluorophore labelled secondary antibody to the bead pellet mixture.
g) Percent adsorption of individual carrier protein was estimated on the basis of fluorescence signal measured by suspension array system i.e. Bioplex200 system.

In the first example of the present disclosure, monoclonal antibodies against carrier proteins of interests were produced using cell hybridoma technique. [Ref: ARC Journal of Immunology and Vaccines; Volume 2, Issue 1, 2017, PP 1-8]. BALB/c (female) mice were immunized with 50µg of carrier proteins along with adjuvant on day 1, 14 and 28. Mice were bled on day 35 to determine humoral immune response against carrier protein of interest and mouse giving good IgG titer against carrier protein of interest were identified. Splenocytes were isolated from the identified mouse and fused with SP2/O-Ag14 cells (mouse myeloma cells) using polyethylene glycol (PEG). Fused cells were selected using HAT (Hypoxanthine Aminopterin Thymidine) medium over a period of 15 days. The supernatant of the fused cells was screened by in-house analytical methods like bead based assay and ELISA to check the presence of antibodies against carrier protein of interest. Limiting dilution (LD1) of the positive clones was performed to select the stable hybrid clones secreting antibodies against carrier protein of interest and Single anti-carrier protein of interest antibody secreting clone in 6 well plates were propagated. Further second limiting dilution (LD2) was performed and selected clones were propagated in 2 litres media for production of MAb (Monoclonal Antibody). Anti-carrier protein Mab was purified using Affinity chromatography (Protein A resins) followed by diafiltration using Phosphate buffer saline. The purified Anti- carrier protein Mab was stored in -20°C till further use.

In one of the aspects of the first example, monoclonal antibodies against tetanus toxoid (TT) were produced using cell hybridoma technique. BALB/c (female) mice were immunized with 50µg of TT along with adjuvant on day 1, 14 and 28. Mice were bled on day 35 to determine humoral immune response against TT and mouse giving good IgG titer against TT were identified. Splenocytes were isolated from the identified mouse immunized with TT and fused with SP2/O-Ag14 cells (mouse myeloma cells) using polyethylene glycol (PEG). Fused cells were selected using HAT (Hypoxanthine Aminopterin Thymidine) medium over a period of 15 days. The supernatant of the fused cells was screened by in-house analytical methods like bead based assay and ELISA to check the presence of antibodies against Tetanus toxoid. Limiting dilution (LD1) of the positive clones was performed to select the stable hybrid clones secreting antibodies against TT and Single anti-TT antibody secreting clone in 6 well plates were propagated. Further second limiting dilution (LD2) was performed and selected clones were propagated in 2 litres media for production of anti-TT Mab. Anti-TT Mab was purified using Affinity chromatography (Protein A resins) followed by diafiltration using Phosphate buffer saline. The purified Anti-TT Mab was stored in -20°C till further use.

It is very well understood that monoclonal antibodies against other carrier proteins like CRM197, diphtheria toxoid (DT), Neisseria meningitidis outer membrane complex, fragment C of tetanus toxoid, pertussis toxoid, protein D of H. influenzae, E. coli LT, E. coli ST, and exotoxin A from Pseudomonas aeruginosa, outer membrane complex c (OMPC), porins, transferrin binding proteins, pneumolysin (Ply), pneumococcal surface protein A (PspA), pneumococcal surface adhesin A (PsaA), pneumococcal PhtD, pneumococcal surface proteins BVH-3 and BVH-11, protective antigen (PA) of Bacillus anthracis and detoxified edema factor (EF) and lethal factor (LF) of Bacillus anthracis, ovalbumin, keyhole limpet hemocyanin (KLH), human serum albumin, bovine serum albumin (BSA) and purified protein derivative of tuberculin (PPD) can also be generated by the person skilled in the art using above mentioned protocol.

In second example of the present disclosure, polyclonal antibodies against CRM197 were produced. [Ref: Hancock and O'Reilly; Synthetic Peptides as Antigens for Antibody Production; Methods in Molecular Biology, vol. 295: Immunochemical Protocols, Third Edition, Humana Press Inc]. CRM197 polyclonal antibodies were generated by immunizing BALB/c (female) mouse with 50µg of CRM197 along with adjuvant on day 1, 14 and 28. The mouse was bled on day 35 to determine humoral response against CRM197. The mouse with good humoral response was selected and terminally bled to isolate the anti-sera. The isolated anti-sera were checked for reactivity with CRM197 and cross reactivity with other carrier proteins for multiplex assay.

It is very well understood that polyclonal antibodies against other carrier proteins like Tetanus toxoid (TT), diphtheria toxoid (DT), Neisseria meningitidis outer membrane complex, fragment C of tetanus toxoid, pertussis toxoid, protein D of H. influenzae, E. coli LT, E. coli ST, and exotoxin A from Pseudomonas aeruginosa, outer membrane complex c (OMPC), porins, transferrin binding proteins, pneumolysin, pneumococcal surface protein A (PspA), pneumococcal surface adhesin A (PsaA), pneumococcal PhtD, pneumococcal surface proteins BVH-3 and BVH-11 , protective antigen (PA) of Bacillus anthracis and detoxified edema factor (EF) and lethal factor (LF) of Bacillus anthracis, ovalbumin, keyhole limpet hemocyanin (KLH), human serum albumin, bovine serum albumin (BSA) and purified protein derivative of tuberculin (PPD) can also be generated by the person skilled in the art using above mentioned protocol.

In third example of the present disclosure, carrier proteins were coupled to polystyrene beads using amine coupling kit (Biorad) procedure. The coupling procedure is applicable to covalently couple the water-soluble proteins ranging in size from 6 to 150 kD via carboxyl groups on the surface of polystyrene beads. The coupling procedure is a two step carbodiimide reaction. Before coupling to protein, the carboxyl groups on the surface of polystyrene beads are activated with EDC (1-ethyl-3-[3-dimethylaminopropyl] carbodiimide hydrochloride) a carbodiimide derivative. EDC (1-ethyl-3-[3-dimethylaminopropyl] carbodiimide hydrochloride) reacts with carboxyl groups on the surface of the polystyrene beads to form an active O-acylisourea intermediate. This unstable intermediate is stabilized in aqueous solutions using S-NHS (N-hydroxysulfosuccinimide). EDC couples S-NHS to the carboxyl group resulting in an S-NHS activated site. Both the carbodiimide's, O-acylisourea intermediate and the S-NHS-ester formed are amine-reactive; however, S-NHS-ester has much greater stability at the physiological pH. These intermediates then react with primary amines of proteins to form amide bonds. The intermediate is hydrolyzed if it fails to react with an amine and the carboxyl is regenerated, releasing an N-unsubstituted urea.

In first aspect of the third example, CRM197 carrier protein was coupled to polystyrene beads using amine coupling kit (Biorad) procedure.

In second aspect of the third example, Tetanus Toxoid carrier protein was coupled to polystyrene beads using amine coupling kit (Biorad) procedure.

In third aspect of the third example, Diphtheria Toxoid carrier protein was coupled to polystyrene beads using amine coupling kit (Biorad) procedure.

It is very well understood that other carrier proteins can also be coupled to polystyrene beads by the person skilled in the art using amine coupling kit (Biorad) procedure.

In fourth example of the present disclosure, carrier proteins were coupled to polystyrene beads using Sulfo-SMCC coupling procedure. The coupling procedure is applicable to covalently couple the water-soluble proteins ranging in size from 6 to 150 kD using sulfo-succinimidyl-4-(N-maleimidomethyl) cyclohexane-1-carboxylate cross linker to the polystyrene beads. The cross linker contains an amine reactive N-hydroxylsuccinimide (NHS ester) and sulfhydryl - reactive maleimide group.

In first aspect of the fourth example, CRM197 carrier protein was coupled to polystyrene beads using Sulfo-SMCC coupling procedure.

In second aspect of the fourth example, Tetanus Toxoid carrier protein was coupled to polystyrene beads using Sulfo-SMCC coupling procedure.

In third aspect of the fourth example, Diphtheria Toxoid carrier protein was coupled to polystyrene beads using Sulfo-SMCC coupling procedure.

It is very well understood that other carrier proteins can also be coupled to polystyrene beads by the person skilled in the art using Sulfo-SMCC coupling procedure.

In the fifth example of the present disclosure, a multiplex bead based competitive inhibition assay was designed to estimate the individual concentration of a carrier protein in a multivalent polysaccharide protein conjugate vaccine composition, wherein two or more carrier proteins are conjugated to different polysaccharides in a vaccine sample.

According to one preferred aspect of the fifth example, a multivalent meningococcal conjugate vaccine (A, C, Y, W & X; conjugated to TT and CRM₁₉₇) composition was analyzed for determining the individual total concentrations of TT and CRM₁₉₇.

In second aspect of the fifth example, a multivalent pneumococcal conjugate vaccine composition was analyzed for determining the individual total concentrations of different carrier proteins, as per protocol mentioned in examples 2 wherein the composition comprises of i) at least 2 polysaccharide protein conjugates having polysaccharide selected from serotypes 1, 2, 3,4,5, 6A, 6B,7F, 8,9V,9F,9N, 12F, 14, 15B, 17F,18C, 19A ,19F,20,22F,23F,33F and 45 and ii) at least two different carrier proteins selected from TT, CRM197, DT and pneumococcal surface adhesin A (PsaA).

In sixth example of the present disclosure, the fluorophore conjugated secondary antibody comprise a fluorophore selected from the group consisting of: indocarbocyanine (C3), indodicarbocyanine (C5), Cy3, Cy3.5, Cy5, Cy5.5, Cy7, Texas Red, Pacific Blue, Oregon Green 488, Alexa fluor-355, Alexa Fluor 488, Alexa Fluor 532, Alexa Fluor 546, Alexa Fluor-555, Alexa Fluor 568, Alexa Fluor 594, Alexa Fluor 647, Alexa Fluor 660, Alexa Fluor 680, Alexa Fluor 700, JOE, Lissamine, Rhodamine Green, BODIPY, fluorescein isothiocyanate (FITC), carboxy-fluorescein (FAM), Allophycocyanin (APC), phycoerythrin (PE), rhodamine, dichlororhodamine (dRhodamine), carboxy tetramethylrhodamine (TAMRA), carboxy-Xrhodamine (ROX), LIZ, VIC, NED, PET, SYBR, PicoGreen, and RiboGreen.

In a preferred aspect of sixth example, the fluorophore conjugated secondary antibody comprises a phycoerythrin fluorophore.

In seventh example of the present disclosure, the readings from suspension array system i.e. Bioplex-200 system were plotted between concentrations on X axis and Fluorescence intensity on Y axis. Using this as standard curve, the individual concentrations of the carrier proteins in a multivalent vaccine formulation were determined.

### TECHNICAL ADVANCEMENTS:

The present disclosure described herein above has several technical advantages when compared to conventional methods:
- Traditional methods like Lowry method, DC method, U.V absorbance at 480nm do not differentiate between two different proteins. However, the present method can specifically determine individual concentration of at least 2 different types of carrier proteins in a sample comprising of multivalent polysaccharide protein conjugate bulk/formulation.
- Up to 90% saving in reagent costs.
- Reduced turnaround time from 21 to 8 hours.
- Low sample volume required.
- Keeps the same work flow as ELISA.
- Multiplexing capability enables to perform multiple experiments simultaneously instead of sequentially.
- Beads generate more reproducible results.

In view of the many possible embodiments to which the principles of the present disclosure may be applied, it should be recognized that the illustrated embodiments are only preferred examples of the present disclosure and should not be taken as limiting the scope of the invention.

### EXAMPLES

### Example 1:

### Coupling of CRM197/TT/DT to polystyrene beads

### [Amine coupling kit Procedure, Bio-Rad]

The coupling procedure involved a two-step carbodiimide reaction. The carboxyl groups on the surface of the polystyrene beads were first activated with a carbodiimide derivative prior coupling to CRM197/TT/DT. EDC (1 -ethyl-3-[3-dimethylaminopropyl] carbodiimide hydrochloride) reacts with carboxyl groups on the surface of the polystyrene beads to form an active O-acylisourea intermediate. This unstable intermediate was stabilized in aqueous solutions using S-NHS (N-hydroxysulfosuccinimide). EDC couples S-NHS to the carboxyl group resulting in an S-NHS activated site. Both the carbodiimide's, O-acylisourea intermediate and the S-NHS-ester formed are amine-reactive; however, S-NHS-ester has much greater stability at the physiological pH. These intermediates then react with primary amines of CRM197/TT/DT to form amide bonds. The intermediate is hydrolyzed if it fails to react with an amine and the carboxyl is regenerated, releasing an N-unsubstituted urea.

The CRM197/TT/DT was coupled to polystyrene bead using following protocol:

### Bead activation:

a) The vial of COOH beads were allowed to come to room temperature for approximately 5 min., and later the beads were vortexed and sonicated for approximately 1-2 min in-order to avoid aggregation and to monodisperse them.
b) 100 µl of mono dispersed COOH beads (1.25 x 106 beads) were added in coupling reaction tube provided with the kit.
c) The beads were centrifuged at 14000 x g for 5 min. The supernatant was carefully removed and discarded from the bead pellet.
d) 100 µl of bead wash buffer was added from the kit. And later the beads were vortexed and sonicated for approximately 10 sec. The beads were centrifuged at 14000 x g for 5 min and again the supernatant was carefully removed and discarded from the bead pellet.
e) The bead pellet was resuspended in 80 µl of bead activation buffer from the kit. Later vortexed for 30 sec, and sonicated by bath sonication for 30 sec.
f) 100 µl each of 50 mg/ml of EDC and S-NHS were prepared by adding accurately weighed 5 mg each of EDC and S-NHS in 100 µl of bead activation buffer in two different tubes, immediately prior to their use.
g) 10 µl of freshly made 50 mg/ml EDC was added in the bead suspension closely followed by 10 µl of freshly made 50 mg/ml of S-NHS.
h) The beads were centrifuged at high speed for 30 sec. The coupling reaction tube was covered with aluminium foil and allowed to rotospin for 20 min at RT with 30 rpm for IX reaction.
i) From the kit 150 µl of PBS, pH 7.4 was added and the activated beads were vortexed at high speed for 10 sec. The Beads were centrifuged at 14000 x g for 5 min and the supernatant was carefully removed and discarded from the bead pellet. This step was repeated one more time.
j) The activated beads were resuspended in 100 µl PBS. The beads were vortexed at medium speed for 30 sec and sonicated by bath sonication for 15 sec.

### CRM197/TT/DT coupling to activated beads:

a) CRM 197/TT/DT (approximately 5 to 15 µg based on optimization experiments) was added to the tube of activated beads.
b) The final volume was adjusted to 500 µl with PBS, pH 7.4. The tube was covered with aluminium foil and the beads were agitated on rotospin at RT for 2 hr with 30 rpm for IX reaction.
k) The coupled beads were centrifuged at 14000 x g for 5 min., and the supernatant was carefully removed and discarded from the bead pellet. This step was repeated one more time.
c) The coupled beads were resuspended with 250 µl of blocking buffer from the kit.
d) And allowed to vortex at medium speed for 15 sec. The tube was later covered with aluminium foil and the beads were agitated on rotospin at RT for 30 min with 30 rpm.
e) The beads were centrifuged at 14000 x g for 5 min., and the supernatant was carefully removed and discarded from the bead pellet.
f) The coupled beads were washed with 500 µl of storage buffer from the kit and centrifuged at 16500 x g for 7 min., and the supernatant was carefully removed and discarded from the bead pellet.
g) The coupled beads were resuspended in 150 µl of storage buffer by vortexing approximately for 1 min.
h) Bead concentration was determined using haemocytometer.

Similarly, Tetanus Toxoid (TT) & Diphtheria Toxoid (DT) carrier protein was coupled to polystyrene beads using amine coupling kit (Biorad) procedure, as described above.

### Example - 2

### Estimation of individual concentration of carrier protein (CRM197/TT) in a multivalent meningococcal polysaccharide - protein conjugate vaccine (MCV5) composition/conjugate bulks by bead based competitive inhibition assay (BBCIA):

a) In this multivalent meningococcal conjugate vaccine (MCV5), polysaccharide from Men-A and Men-X are conjugated to TT, whereas the polysaccharide from Men-C, Men-W and Men-Y are conjugated to CRM197.
b) COA of each conjugate vaccine provides accurate details of Polysaccharide to protein ratio of individual conjugate. Further the theoretical concentration of total CRM197 & TT in final vaccine formulation was calculated on the basis of colorimetric value of CRM197 & TT in individual conjugate bulk.
c) Take 1 ml of working standard (MCV5 formulation) in 1 ml of microfuge tube. Dilute it to the final concentration of 2.72 µg/mL of CRM content and 2 µg/mL of TT content (depending upon Ps/Protein ratio of respective conjugates in final formulation) using luminex assay buffer.

Further dilutions were carried out as per following table.

**Table 1**

| **Preparation of standards by dilution with assay buffer** | | | | |
|---|---|---|---|---|
| Vaccine formulation Standard | Working standard (µL) | Luminex assay buffer (µL) | Concentration (µg/mL) | |
| | | | CRM | TT |
| S1 | 250 µL working stock solution | - | 2.72 | 2 |
| S2 | 125 µL of S1 solution | 125 | 1.36 | 1 |
| S3 | 125 µL of S2 solution | 125 | 0.68 | 0.5 |
| S4 | 125 µL of S3 solution | 125 | 0.34 | 0.25 |
| S5 | 125 µL of S4 solution | 125 | 0.17 | 0.125 |
| S6 | 125 µL of S5 solution | 125 | 0.085 | 0.0625 |
| S7 | 125 µL of S6 solution | 125 | 0.0425 | |

**The total concentration of individual carrier protein was determined using following protocol:**
a) 100 µl of the standards were transferred to respective adjacent columns in the same plate from the above series of dilution.
b) A mixture of 1:20000 diluted anti CRM mice sera and 1:50000 diluted anti TT MAb were prepared using luminex assay buffer. 100 µl of this antibody solution were added in to each respective well of standards and sample.
c) The plate was incubated at 37°C for 1 hour with shaking at 150 rpm for pre-adsorption.
d) Prewet the required wells of multiscreen filter plate with 100 µl of luminex assay buffer for 1-2 minutes and aspirate the plate using Vacuum manifold.
e) A mixture of a required volume of CRM and TT coupled beads were prepared in luminex assay buffer to obtain ∼4000 beads of each carrier protein per 50µl of final suspension. 4000 beads per 50µl were added in each required well of filter plate. The filter plate was aspirated using Vacuum manifold. (Caution: Beads are photosensitive, utmost care should be taken to avoid exposure of samples to direct light).
f) 50µl of pre-incubated standards/samples from dilution plate of step (c) were added in duplicates on filter plate containing beads from step (e) with positive and negative control as per assay plate template planned. The plates were incubated at 37°C for 1 hour with shaking at 150 rpm in dark.
g) The plates were washed with 100ul of luminex assay buffer followed by aspiration using vacuum manifold. This step was repeated 3 times.
h) A mixture of required volume of Anti-mouse IgG-phycoerythrin (Anti-M-PE) secondary antibody (fluorescent reporter) with a final dilution of 1:250 was prepared and 50 µl of this was added in each well containing standards and samples. The plates were incubated at 37°C for 30 minutes with shaking at 150 rpm in dark.
i) The plates were washed three times following the procedure mentioned in bead washing step.
j) 100ul of luminex assay buffer was added to each well and further the plate was read on Bio-Plex 200 system.
k) Beads are coloured internally with two different fluorescent dyes (red and infrared). Ten different concentrations of red and infrared dyes are used to generate distinct bead regions. Each bead region is conjugated to respective carrier protein. Dyed beads are pushed through a detection chamber in a single file. The red classification laser (635 nm) interrogates internal dye to identify bead regions. The green reporter laser (532 nm) interrogates fluorescent reporter to measure analyte concentration in that bead regions. Individual concentration of carrier protein was estimated on the basis of fluorescence signal measured in that distinct bead region by suspension array system i.e. Bioplex200 system.

### Example 3:

### Estimation of individual concentration of carrier protein (CRM197/TT/DT) in a multivalent pneumococcal polysaccharide - protein conjugate vaccine (PCV) composition/conjugate bulks by bead based competitive inhibition assay (BBCIA):

a) In this multivalent pneumococcal polysaccharide - protein conjugate vaccine (PCV) composition, polysaccharide selected from serotypes 1, 2, 3, 4, 5, 6A, 6B, 7F, 8, 9V, 9F, 9N, 12F, 14, 15B, 17F, 18C, 19A, 19F, 20, 22F, 23F, 33F and 45 are conjugated to at least two different carrier proteins selected from TT, CRM197, DT and pneumococcal surface adhesin A (PsaA) incorporated from PCT Patent Application No. PCT/IB2016/053265 entitled "Methods for improving the adsorption of polysaccharide-protein conjugates and multivalent vaccine formulation obtained thereof', filed June 3, 2016.
b) COA of each conjugate vaccine provides accurate details of Polysaccharide to protein ratio of individual conjugate. Further the theoretical concentration of total CRM197, DT & TT in final vaccine formulation was calculated on the basis of colorimetric value of CRM197, DT & TT in individual conjugate bulk.
c) Take 1 ml of working standard (PCV formulation) in 1 ml of microfuge tube. Dilute it to the final concentration of 3200 ng/mL of CRM content, 175 ng/mL of TT content and 444 ng/ml of DT (depending upon Ps/Protein ratio of respective conjugates in final formulation) using luminex assay buffer.

Further dilutions were carried out as per following table.

**Table 2**

| **Preparation of standards by dilution with assay buffer** | | | | | |
|---|---|---|---|---|---|
| Vaccine formulation Standard | Working standard (µL) | Luminex assay buffer (µL) | Concentration (ng/mL) | | |
| | | | CRM | TT | DT |
| S1 | 250 µL working stock solution | - | 3200 | 175 | 444 |
| S2 | 125 µL of S1 solution | 125 | 1600 | 87.5 | 222 |
| S3 | 125 µL of S2 solution | 125 | 800 | 43.8 | 111 |
| S4 | 125 µL of S3 solution | 125 | 400 | 21.9 | 55.5 |
| S5 | 125 µL of S4 solution | 125 | 200 | 10.9 | 27.8 |
| S6 | 125 µL of S5 solution | 125 | 100 | 5.5 | 13.9 |
| S7 | 125 µL of S6 solution | 125 | 50 | | 6.9 |
| S8 | 125 µL of S7 solution | 125 | 25 | | 3.5 |

**The total concentration of individual carrier protein was determined using following protocol:**
1) 100 µl of the standards were transferred to respective adjacent columns in the same plate from the above series of dilution.
m) A mixture of 1:20000 diluted anti CRM mice sera and 1:50000 diluted anti TT MAb were prepared using luminex assay buffer. 100 µl of this antibody solution were added in to each respective well of standards and sample.
n) The plate was incubated at 37°C for 1 hour with shaking at 150 rpm for pre-adsorption.
o) Prewet the required wells of multiscreen filter plate with 100 µl of luminex assay buffer for 1-2 minutes and aspirate the plate using Vacuum manifold.
p) A mixture of a required volume of CRM and TT coupled beads were prepared in luminex assay buffer to obtain ∼4000 beads of each carrier protein per 50µl of final suspension. 4000 beads per 50µl were added in each required well of filter plate. The filter plate was aspirated using Vacuum manifold. (Caution: Beads are photosensitive, utmost care should be taken to avoid exposure of samples to direct light).
q) 50µl of pre-incubated standards/samples from dilution plate of step (c) were added in duplicates on filter plate containing beads from step (e) with positive and negative control as per assay plate template planned. The plates were incubated at 37°C for 1 hour with shaking at 150 rpm in dark.
r) The plates were washed with 100ul of luminex assay buffer followed by aspiration using vacuum manifold. This step was repeated 3 times.
s) A mixture of required volume of Anti-mouse IgG-phycoerythrin (Anti-M-PE) secondary antibody (fluorescent reporter) with a final dilution of 1:250 was prepared and 50 µl of this was added in each well containing standards and samples. The plates were incubated at 37°C for 30 minutes with shaking at 150 rpm in dark.
t) The plates were washed three times following the procedure mentioned in bead washing step.
u) 100ul of luminex assay buffer was added to each well and further the plate was read on Bio-Plex 200 system.
v) Beads are coloured internally with two different fluorescent dyes (red and infrared). Ten different concentrations of red and infrared dyes are used to generate distinct bead regions. Each bead region is conjugated to respective carrier protein. Dyed beads are pushed through a detection chamber in a single file. The red classification laser (635 nm) interrogates internal dye to identify bead regions. The green reporter laser (532 nm) interrogates fluorescent reporter to measure analyte concentration in that bead regions. Individual concentration of carrier protein was estimated on the basis of fluorescence signal measured in that distinct bead region by suspension array system i.e. Bioplex200 system.

### EXAMPLE 4

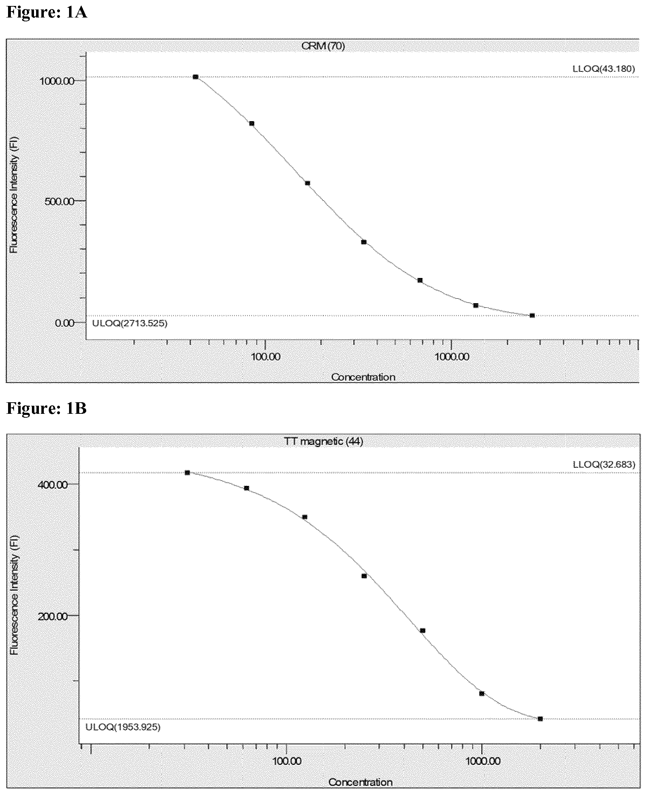

### Standard curve for estimation of LLOD, LLOQ and ULOQ of CRM 197 & TT Carrier Protein in MCV5 composition: (Refer: Table 1 and Figure 1A & 1B)

Different dilutions of standard formulation were assayed by multiplexed BBCIA to obtain standard Curve

### Inference:

Lower limit of detection (LLOD): for CRM; 0.021µg/ml, for TT; 0.0156µg/ml
Lower limit of quantification (LLOQ): for CRM; 0.042µg/ml, for TT; 0.031µg/ml
Upper limit of quantification (ULOQ): for CRM; 2.72µg/ml, for TT; 2µg/ml

### EXAMPLE 5

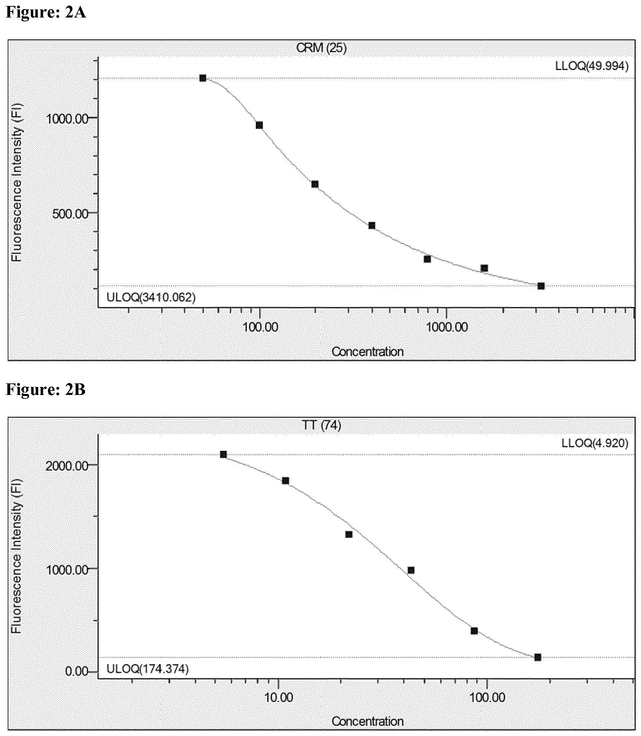

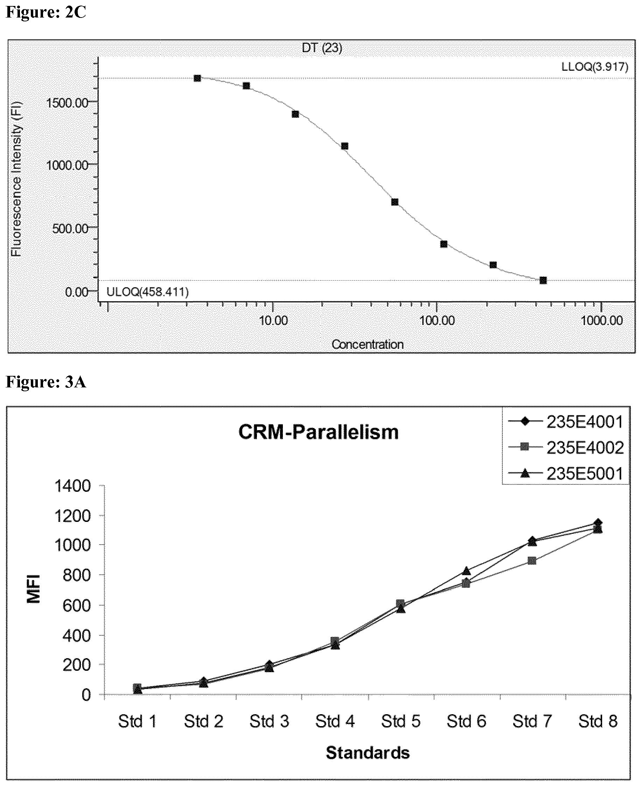

### Standard curve for estimation of LLOD, LLOQ and ULOQ of CRM 197, DT & TT Carrier Protein in PCV composition: (Refer: Table 2 and Figure 2A, 2B & 2C)

Different dilutions of standard formulation were assayed by multiplexed BBCIA to obtain standard Curve

### Inference:

Lower limit of detection (LLOD): for CRM; 0.025µg/ml, for TT; 0.0027µg/ml and DT: 0.0017ug/ml
Lower limit of quantification (LLOQ): for CRM; 0.050µg/ml, for TT; 0.005µg/ml and DT: 0.0034ug/ml
Upper limit of quantification (ULOQ): for CRM197; 3.2 µg/ml, for TT; 0.175 µg/ml and for DT; 0.444 ug/ml

### EXAMPLE 6:

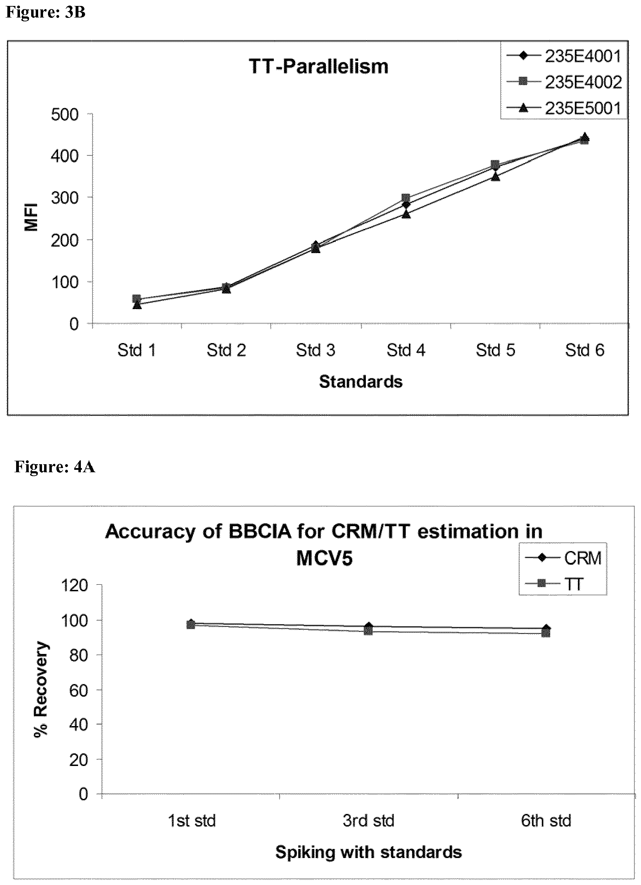

### CRM197-TT Multiplexed BBCIA Parallelism analysis in MCV5 composition to determine assay suitability: (Refer: Figure 3A & 3B)

Different MCV5 formulations were determined for parallelism at similar protein concentrations in multiplexed BBCIA

**Table 2**

| **Serogroup** | **Concentration µg/ml** | | |
|---|---|---|---|
| | **235E4001 Formulation** 1 | **235E4002 Formulation 2** | **235E5001 Formulation 3** |
| Men A | 20.41 | 19.23 | 19.23 |
| Men X | 21.74 | 22.22 | 19.6 |
| **Total TT** | **42.15** | **41.45** | **38.83** |
| Men C | 18.51 | 19.23 | 16.66 |
| Men Y | 24.39 | 24.39 | 12.98 |
| Men W | 14.7 | 16.66 | 14.92 |
| **Total CRM** | **57.6** | **60.28** | **44.56** |

The theoretical concentration of total CRM197 and TT in 235E4001 formulation, (on the basis of colorimetric value of CRM197 and TT in monovalent conjugate bulk) was found to be 57.6 and 42.15 µg/ml respectively. The formulation was diluted 21.1 fold, wherein the final concentration of CRM197 and TT would become 2.72 µg/ml and 2 µg/ml respectively in 235E4001 formulation. Similarly, 235E4002 & 235E5001 formulation were diluted respectively to obtain similar carrier protein concentration.

### Inference:

Parallelism observed between different pentavalent meningococcal conjugate vaccine formulations having similar protein concentrations in multiplexed assay hence determines assay suitability.

### EXAMPLE 7:

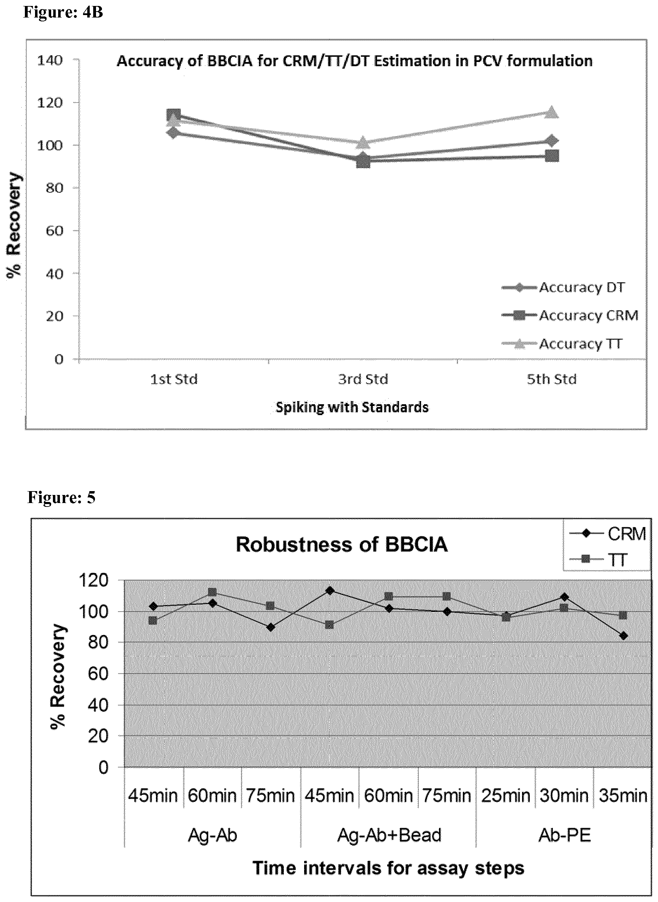

**Accuracy of Multiplexed BBCIA:** (Refer: Table 1, Table 2, and Figure 4A & 4B) Accuracy of multiplexed BBCIA was determined by spiking MCV5 formulation with 1^{st}, 3^{rd} and 6^{th} standards and by spiking PCV formulation with 1^{st}, 3^{rd} and 5^{th} standards. Spike recovery of CRM197, DT and TT in multiplexed BBCIA was checked.

**Inference:** Spike recovery of respective carrier protein in MCV5 and PCV formulation was observed to be in between 100 ± 20% in the assay which suggests that above mentioned method is accurate.

### EXAMPLE 8:

### Robustness of Multiplexed BBCIA for CRM/TT estimation in MCV5 formulation (Refer: Figure 5)

To determine robustness of multiplexed BBCIA, incubation time of all the steps of the assay were deviated from optimized incubation time.

Optimized incubation time of different steps:
1) Ag-Ab reaction: 60 min
2) Ag-Ab with Ag coated beads: 60 min
3) Beads with Phycoerythrene-Ab: 30 min

**Inference:** No significant difference in concentration of CRM/TT in final vaccine formulation was observed after deviating from optimized incubation time of different steps in the assay. This indicates that BBCIA is very robust for estimation of CRM/TT in pentavalent meningococcal conjugate vaccine.

### EXAMPLE 9:

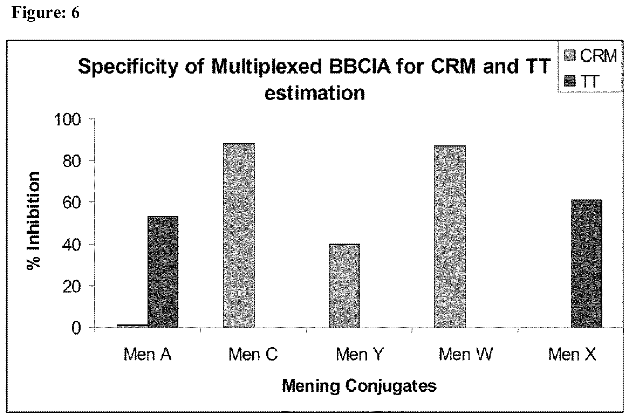

### Specificity of Multiplexed BBCIA for CRM/TT estimation in MCV5 formulation (Refer: Figure 6)

- Men A and Men X Polysaccharide are conjugated with TT. Men C, Men Y and Men W Polysaccharide are conjugated with CRM.
- Men A and Men X conjugate should not react with anti TT antibody and Men C, Men Y and Men W conjugate should not react with anti CRM antibody.
- Hence, competitive inhibition would be negligible for Men A and X conjugates for CRM and vice versa for TT.
- Similarly, competitive inhibition would be negligible for Men C, Y and W conjugate for TT and vice versa for CRM.
- 1µg/ml of MnPs conjugates were incubated with either anti TT antibody to check the specificity of multiplexed BBCIA for CRM/TT estimation in below mentioned expt.
**Inference:** Multiplexed BBCIA was found to be specific for respective carrier proteins.

## Claims

1. A method for estimation of individual protein from a multivalent polysaccharide protein conjugate sample containing at least two different types of protein, wherein the protein is a carrier protein and wherein the carrier protein is selected from the group comprising Tetanus toxoid (TT), Diphtheria toxoid (DT) and CRM197, comprising the steps of:
a) covalent coupling of microspheres to protein of interest to be estimated, wherein the coupling of protein to microsphere is carried out using carbodiimide conjugation chemistry and wherein the microspheres are polystyrene beads;
b) contacting a known concentration of anti - protein antibody to the sample containing multivalent polysaccharide-protein conjugate, free polysaccharide and free protein that binds to the epitope of protein;
c) further separating the free or unbound anti - protein antibody by contacting a known concentration of protein coupled to microspheres to the sample of step (b) wherein the free or unbound anti - protein antibody binds to the epitope of protein coupled to microspheres;
d) contacting the separated free or unbound anti - protein antibody bound to protein coupled microspheres from the sample of step (c) to a fluorophore labelled secondary antibody;
e) measuring the percent adsorption of carrier protein on the basis of fluorescence signal measured by suspension array system.

2. The method of claim 1, wherein the said anti - protein antibody is against Tetanus toxoid (TT), Diphtheria toxoid (DT) or CRM197.

3. The method of claim 1, wherein the said anti - protein antibody is a monoclonal antibody.

4. The method of claim 1, wherein the said anti - protein antibody is a polyclonal antibody.

5. The method of claim 1, wherein said protein to be estimated is conjugated to polysaccharide selected from the group consisting of Helicobacter pylori, Chlamydia pneumoniae, Chlamydia trachomatis, Ureaplasma urealyticum, Mycoplasma pneumoniae, Staphylococcus spp., Staphylococcus aureus, Streptococcus spp., Streptococcus pyogenes, Streptococcus pneumoniae, Streptococcus viridans, Enterococcus faecalis, Neisseria meningitidis, Neisseria gonorrhoeae, Bacillus anthracis, Salmonella spp., Salmonella typhi, Vibrio cholera, Pasteurella pestis, Pseudomonas aeruginosa, Campylobacter spp., Campylobacter jejuni, Clostridium spp., Clostridium difficile, Mycobacterium spp., Mycobacterium tuberculosis, Treponema spp., Borrelia spp., Borrelia burgdorferi, Leptospira spp., Hemophilus ducreyi, Corynebacterium diphtheria, Bordetella pertussis, Bordetella parapertussis, Bordetella bronchiseptica, Haemophilus influenzae, Escherichia coli, Shigella spp., Erlichia spp., and Rickettsia spp.

6. The method of claim 5, wherein said protein to be estimated is conjugated to N. meningitidis polysaccharide of serotype selected from the group consisting of A, B, C, D, W135, X, Y, Z and 29E.

7. The method of claim 5, wherein said protein to be estimated is conjugated to Streptococcus pneumoniae polysaccharide of serotype selected from the group consisting of: 1, 2, 3, 4, 5,6A, 6B, 7F, 8, 9N, 9V, 10A, 11 A, 12F, 14, 15B, 17F, 18C, 19A, 19F, 20, 22F, 23F, and 33F.

8. The method according to claim 1, wherein the fluorophore labelled secondary antibody comprise a fluorophore selected from the group consisting of: indocarbocyanine (C3), indodicarbocyanine (C5), Cy3, Cy3.5, Cy5, Cy5.5, Cy7, Rhodamine Green, fluorescein isothiocyanate (FITC), carboxy-fluorescein (FAM), Allophycocyanin (APC), phycoerythrin (PE), rhodamine, dichlororhodamine (dRhodamine), carboxy tetramethylrhodamine (TAMRA), carboxy-Xrhodamine (ROX).

9. The method according to claim 8, wherein the secondary antibody is labelled with a phycoerythrin fluorophore.

10. The method according to any of the previous claims, wherein the suspension array system contains a bead array reader.

11. A method of any of the preceding claims, wherein the sample being assayed for protein consists of (a) a conjugate of (i) the capsular saccharide of serogroup A N. meningitidis and (ii) tetanus toxoid; (b) a conjugate of (i) capsular saccharide of serogroup C N. meningitidis and (ii) CRM197; (c) a conjugate of (i) capsular saccharide of serogroup Y N. meningitidis and (ii) CRM197; (d) a conjugate of (i) capsular saccharide of serogroup W135 N. meningitidis and (ii) CRM197; and (e) a conjugate of (i) capsular saccharide of serogroup X N. meningitidis and (ii) tetanus toxoid.

## Patentansprüche

1. Ein Verfahren zur Abschätzung eines individuellen Proteins aus einer Probe eines multivalenten Polysaccharid-Protein-Konjugats, die wenigstens zwei unterschiedliche Typen von Protein enthält, wobei das Protein ein Trägerprotein ist und wobei das Trägerprotein ausgewählt ist aus der Gruppe, umfassend Tetanus-Toxoid (TT), Diphtherie-Toxoid (DT) und CRM197, umfassend die Schritte:
a) das kovalente Verbinden von Mikrokügelchen mit dem Protein von Interesse, das bestimmt werden soll, wobei das Verbinden des Proteins mit dem Mikrokügelchen unter Anwendung der Karbodiimid-Konjugations-Chemie durchgeführt wird und wobei die Mikrokügelchen Polystyrolkügelchen sind,
b) In-Kontakt-Bringen einer bekannten Konzentration eines Anti-Protein-Antikörpers, der an das Epitop des Proteins bindet, mit der Probe, die das multivalente Polysaccharid-Protein-Konjugat, freies Polysaccharid und freies Protein enthält,
c) des Weiteren das Abtrennen des freien oder ungebundenen Anti-Protein-Antikörpers durch In-Kontakt-Bringen einer bekannten Konzentration von an Mikrokügelchen gebundenem Protein zu der Probe aus Schritt (b), wobei der freie oder ungebundene Anti-Protein-Antikörper an das Epitop des an Mikrokügelchen gebundenen Proteins bindet,
d) In-Kontakt-Bringen des abgetrennten, an das an Mikrokügelchen gebundene Protein gebundenen freien oder ungebundenen Anti-Protein-Antikörpers aus der Probe aus Schritt (c) an einen Fluorophormarkierten Sekundärantikörper,
e) Messen der prozentualen Adsorption des Trägerproteins auf Basis des Fluoreszenz-Signals, das durch ein Suspensions-Array-System gemessen wird.

2. Das Verfahren gemäß Anspruch 1, wobei der Anti-Protein-Antikörper einer gegen Tetanus-Toxoid (TT), Diphterie-Toxoid (DT) oder CRM197 ist.

3. Das Verfahren gemäß Anspruch 1, wobei der Anti-Protein-Antikörper ein monoklonaler Antikörper ist.

4. Das Verfahren gemäß Anspruch 1, wobei der Anti-Protein-Antikörper ein polyklonaler Antikörper ist.

5. Das Verfahren gemäß Anspruch 1, wobei das Protein, das abgeschätzt werden soll, an ein Polysaccharid gebunden ist, ausgewählt aus der Gruppe, bestehend aus Helicobacter pylori, Chlamydia pneumoniae, Chlamydia trachomatis, Ureaplasma urealyticum, Mycoplasma pneumoniae, Staphylococcus spp., Staphylococcus aureus, Streptococcus spp., Streptococcus pyogenes, Streptococcus pneumoniae, Streptococcus viridans, Enterococcus faecalis, Neisseria meningitidis, Neisseria gonorrhoeae, Bacillus anthracis, Salmonella spp., Salmonella typhi, Vibrio cholera, Pasteurella pestis, Pseudomonas aeruginosa, Campylobacter spp., Campylobacter jejuni, Clostridium spp., Clostridium difficile, Mycobacterium spp., Mycobacterium tuberculosis, Treponema spp., Borrelia spp., Borrelia burgdorferi, Leptospira spp., Hemophilus ducreyi, Corynebacterium diphtheria, Bordetella pertussis, Bordetella parapertussis, Bordetella bronchiseptica, Haemophilus influenzae, Escherichia coli, Shigella spp., Erlichia spp., und Rickettsia spp.

6. Das Verfahren gemäß Anspruch 5, wobei das Protein, das abgeschätzt werden soll, an ein N. meningitidis Polysaccharid des Serotyps, ausgewählt aus der Gruppe, bestehend aus A, B, C, D, W135, X, Y, Z und 29E konjugiert ist.

7. Das Verfahren gemäß Anspruch 5, wobei das Protein, das abgeschätzt werden soll, an ein Streptococcus pneumoniae Polysaccharid des Serotyps, ausgewählt aus der Gruppe, bestehend aus: 1, 2, 3, 4, 5, 6A, 6B, 7F, 8, 9N, 9V, 10A, 11A, 12F, 14, 15B, 17F, 18C, 19A, 19F, 20, 22F, 23F, und 33F konjugiert ist.

8. Das Verfahren gemäß Anspruch 1, wobei der Fluorophor-markierte Sekundärantikörper ein Fluorophor ausgewählt aus der Gruppe, bestehend aus: Indocarbocyanin (C3), Indodicarbocyanin (C5), Cy3, Cy3.5, Cy5, Cy5.5, Cy7, Rhodamin Green, Fluoresceinisothiocyanat (FITC), Carboxy-Fluorescein (FAM), Allophycocyanin (APC), Phycoerythrin (PE), Rhodamine, Dichlorrhodamin (dRhodamin), Carboxy-Tetramethylrhodamin (TAMRA), Carboxy-Xrhodamin (ROX) umfasst.

9. Das Verfahren gemäß Anspruch 8, wobei der Sekundärantikörper mit einem Phycoerythrin-Fluorophor markiert ist.

10. Das Verfahren gemäß einem der vorhergehenden Ansprüche, wobei das Suspensions-Array-System einen Bead-Array-Reader enthält.

11. Ein Verfahren gemäß einem der vorhergehenden Ansprüche, wobei die Probe, die auf das Protein untersucht wird, aus (a) einem Konjugat aus (i) dem kapsulären Saccharid der Serogruppe A N. meningitidis und (ii) Tetanus-Toxoid, (b) einem Konjugat aus (i) kapsulärem Saccharid der Serogruppe C N. meningitidis und (ii) CRM197, (c) einem Konjugat aus (i) kapsulärem Saccharid der Serogruppe Y N. meningitidis und (ii) CRM197, (d) einem Konjugat aus (i) kapsulärem Saccharid der Serogruppe W135 N. meningitidis und (ii) CRM197, und (e) einem Konjugat aus (i) kapsulärem Saccharid der Serogruppe X N. meningitidis und (ii) Tetanus-Toxoid besteht.

## Revendications

1. Procédé d'estimation d'une protéine individuelle à partir d'un échantillon de conjugué polysaccharide-protéine multivalent contenant au moins deux types différents de protéine, dans lequel la protéine est une protéine de support et dans lequel la protéine de support est sélectionnée dans le groupe comprenant une anatoxine tétanique (TT), une anatoxine diphtérique (DT) et CRM197, comprenant les étapes suivantes :
a) le couplage par liaison covalente de microsphères à une protéine d'intérêt à estimer, dans lequel le couplage d'une protéine à des microsphères est réalisé en utilisant une chimie de conjugaison par carbodiimide et dans lequel les microsphères sont des billes de polystyrène ;
b) la mise en contact d'une concentration connue d'un anticorps anti-protéine avec l'échantillon contenant un conjugué polysaccharide-protéine multivalent, un polysaccharide libre et une protéine libre qui se lie à l'épitope d'une protéine ;
c) en outre la séparation de l'anticorps anti-protéine libre ou non lié par la mise en contact d'une concentration connue d'une protéine couplée à des microsphères avec l'échantillon de l'étape (b) dans lequel l'anticorps anti-protéine libre ou non lié se lie à l'épitope d'une protéine couplée à des microsphères ;
d) la mise en contact de l'anticorps anti-protéine libre ou non lié séparé lié à une protéine couplée à des microsphères provenant de l'échantillon de l'étape (c) avec un anticorps secondaire marqué par fluorophore;
e) la mesure du pourcentage d'adsorption de la protéine de support sur la base d'un signal de fluorescence mesuré par un système de réseau en suspension.

2. Procédé selon la revendication 1, dans lequel ledit anticorps anti-protéine est dirigé contre une anatoxine tétanique (TT), une anatoxine diphtérique (DT) ou CRM197.

3. Procédé selon la revendication 1, dans lequel ledit anticorps anti-protéine est un anticorps monoclonal.

4. Procédé selon la revendication 1, dans lequel ledit anticorps anti-protéine est un anticorps polyclonal.

5. Procédé selon la revendication 1, dans lequel ladite protéine à estimer est conjuguée à un polysaccharide sélectionné dans le groupe consistant en Helicobacter pylori, Chlamydia pneumoniae, Chlamydia trachomatis, Ureaplasma urealyticum, Mycoplasma pneumoniae, Staphylococcus spp., Staphylococcus aureus, Streptococcus spp., Streptococcus pyogenes, Streptococcus pneumoniae, Streptococcus viridans, Enterococcus faecalis, Neisseria meningitidis, Neisseria gonorrhoeae, Bacillus anthracis, Salmonella spp., Salmonella typhi, Vibrio choiera, Pasteurella pestis, Pseudomonas aeruginosa, Campylobacter spp., Campylobacter jejuni, Clostridium spp., Clostridium difficile, Mycobacterium spp., Mycobacterium tuberculosis, Treponema spp., Borrelia spp., Borrelia burgdorferi, Leptospira spp., Hemophilus ducreyi, Corynebacterium diphtheria, Bordetella pertussis, Bordetella parapertussis, Bordetella bronchiseptica, Haemophilus influenzae, Escherichia coli, Shigella spp., Erlichia spp., et Rickettsia spp.

6. Procédé selon la revendication 5, dans lequel ladite protéine à estimer est conjuguée à un polysaccharide de N. meningitidis de sérotype sélectionné dans le groupe consistant en A, B, C, D, W135, X, Y, Z et 29E.

7. Procédé selon la revendication 5, dans lequel ladite protéine à estimer est conjuguée à un polysaccharide de Streptococcus pneumoniae de sérotype sélectionné dans le groupe consistant en : 1, 2, 3, 4, 5, 6A, 6B, 7F, 8, 9N, 9V, 10A, 11A, 12F, 14, 15B, 17F, 18C, 19A, 19F, 20, 22F, 23F, et 33F.

8. Procédé selon la revendication 1, dans lequel l'anticorps secondaire marqué par fluorophore comprend un fluorophore sélectionné dans le groupe consistant en : l'indocarbocyanine (C3), l'indodicarbocyanine (C5), Cy3, Cy3.5, Cy5, Cy5.5, Cy7, la rhodamine verte, l'isothiocyanate de fluorescéine (FITC), la carboxyfluorescéine (FAM), l'allophycocyanine (APC), la phycoérythrine (PE), la rhodamine, la dichlororhodamine (dRhodamine), la carboxy tétraméthylrhodamine (TAMRA), la carboxy-Xrhodamine (ROX).

9. Procédé selon la revendication 8, dans lequel l'anticorps secondaire est marqué avec un fluorophore phycoérythrine.

10. Procédé selon l'une quelconque des revendications précédentes, dans lequel le système de réseau en suspension contient un lecteur de réseau de billes.

11. Procédé selon l'une quelconque des revendications précédentes, dans lequel l'échantillon analysé pour une protéine consiste en (a) un conjugué (i) du saccharide capsulaire de N. meningitidis de sérogroupe A et (ii) d'une anatoxine tétanique ; (b) un conjugué (i) de saccharide capsulaire de N. meningitidis de sérogroupe C et (ii) de CRM197 ; (c) un conjugué (i) de saccharide capsulaire de N. meningitidis de sérogroupe Y et (ii) de CRM197 ; (d) un conjugué (i) de saccharide capsulaire de N. meningitidis de sérogroupe W135 et (ii) de CRM197 ; et (e) un conjugué (i) de saccharide capsulaire de N. meningitidis de sérogroupe X et (ii) d'une anatoxine tétanique.
